# EUROPEAN PATENT SPECIFICATION

(11) **EP 0 560 981 B1**
(45) Date of publication and mention of the grant of the patent: **22.07.1998**
(21) Application number: 91907702.4
(22) Date of filing: 18.04.1991
(51) Int. Cl.: C02F 3/10, C02F 3/28, C02F 11/04, B09B 3/00

(54) **SYSTEM FOR TREATING ORGANIC WASTES AND WASTE WATER**
ANLAGE ZUR BEHANDLUNG VON ORGANISCHEN ABFÄLLEN UND ABWASSER
SYSTEME DE TRAITEMENT DES DECHETS ORGANIQUES ET DES EAUX RESIDUAIRES

(30) Priority: 19.04.1990 JP 103700/90
(43) Date of publication of application: 22.09.1993
(73) Proprietor: MORI-GUMI CO. LTD., Osaka 541 (JP); TAGUCHI, Akira, Osaka 577 (JP)
(72) Inventor: TAGUCHI, Akira, 8-14, Kamikosaka 2-chome, Osaka 577 (JP)
(74) Representative: Carter, Caroline Ann
(86) International application number: JP9100515
(87) International publication number: WO9116268

(56) References cited:
- EP-A- 96 170
- EP-A- 159 535
- EP-A- 162 339
- EP-A- 213 691
- DE-A- 3 248 703
- FR-A- 2 612 914
- GB-A- 2 017 075
- JP-A- 1 004 300
- JP-A-59 032 915
- JP-A-60 244 396
- JP-A-61 158 786
- DATABASE WPI Section Ch, Week 2188, Derwent Publications Ltd., London, GB; Class D15, AN 88-143478

## Description

### TECHNICAL FIELD

This invention relates to an improvement of a treating system for treating such organic wastes and liquid waste as excrement, kitchen garbage and organic industrial liquid waste, and more particularly, to an improvement of a methane fermentation tank for treating organic solid or liquid waste utilizing methane fermentation, filter media and an stirring device for stirring the inside of the tank.

### BACKGROUND ART

There have been many developments in the field of anaerobic treatment of organic solid and liquid waste, including a simple single-tank system requiring 20 to 40 days for the completion of treatment, a double-tank system with higher treatment efficiency, a system using a fixed or fluid filtering bed, a system wherein bacteria accumulate in large quantities in a reaction tank in a granular or integrated solid form, which has drastically increased treatment efficiency, and a system wherein bacteria are allowed to float and multiply so that their fissiparous membranes are utilized. The efficiency of anaerobic treatment has thus increased remarkably.

However, systems of high efficiency are generally limited to treatment of specific organic matter. It is also considered difficult in general to treat organic matter in low content, and in many cases, advanced techniques are required for the adaptation of bacteria to the systems and for their successful operation.

### DISCLOSURE OF INVENTION

It is therefore an object of the present invention to provide a simple and highly efficient treating system for the treatment of organic solid and liquid waste which is capable of treating not only specific organic waste of high content but a wide range of various organic waste, including home waste water containing organic substances of low content, urban sewage, combined sewage (mixture of liquid waste consisting of flush toilet sewage and home waste water), home solid waste such as leftover food and other food waste, waste water mixed with disposal-processed garbage, human waste, and livestock and industrial waste water.

The inventor of this invention has previously provided in JP-A-64 4300 a treating system for organic solid and liquid waste to attain similar objects. The present invention attempts to improve said treating system, treating tank used in said system, filter media used in said tank and automatic gas-lift stirring device used to stir contents in the treating tank, so that anaerobic treatment is accomplished with higher efficiency.

There are three types of embodiments of the treating tank to be used in the treating system in accordance with the present invention, that is, a horizontal zigzag flow tank, a vertical zigzag flow tank and a vertical alternate flow tank.

Accordingly, the present invention provides a treating system for organic solid and liquid waste comprising:
a horizontal zigzag flow treating tank which comprises a sloping bottom and a plurality of chambers formed by horizontally dividing the inside of the tank with partitions disposed in a zigzag fashion with each partition orientated differently from an adjacent partition, the chambers being connected to one another in a horizontal zigzag fashion,
filter media to hold bacteria installed in each chamber in a standing position, the filter media being composed of a core element and a plurality of thin tapes of synthetic resin extending radially around the core element, the filter media being installed in such a manner that, in use of the system, the tapes float in liquid waste to be treated with their ends slightly touching those of adjacent tapes,
and an automatic stirring device having a gas chamber connected through valves to a methane gas outlet provided at an upper portion of the tank and to methane gas inlet for stirring purposes at the bottom of the tank, the stirring device comprising a water tank holding a prescribed amount of liquid, a stirring tower with an open top connected to the top of the water tank, the gas chamber being situated in the water tank and having an open bottom through which the gas chamber is connected to the inside of the water tank and the stirring tower, and detecting means for generating control signals to close a valve to the methane gas outlet and open a valve to the methane gas inlet when detecting a prescribed upper level of the liquid rising in said stirring tower due to gas pressure in the gas chamber,
the treating tank being such that substances to be treated can be received at the upper end of the slope and treated substances can be discharged from the lower end of the slope.

The invention also provides a treating system for organic solid and liquid waste comprising:
a vertical zigzag flow treating tank which comprises a plurality of chambers formed by vertically dividing the inside of the tank with partitions extending in opposite directions in turn from the inner wall of the tank with a gap provided at each end so that the chambers are connected to one another in a vertical zigzag fashion whereby fluids to be treated flow substantially parallel to the partitions,
filter media to hold bacteria installed in each chamber in a lying position, the filter media being composed of a core element and a plurality of thin tapes of synthetic resin extending radially around the core element, the filter media being installed in such a manner that, in use of the system, the tapes float in liquid waste to be treated with their ends slightly touching those of adjacent tapes,
and an automatic stirring device having a gas chamber connected through valves to a methane gas outlet provided at an upper portion of the tank and to a methane gas inlet for stirring purposes at the bottom of the tank, the stirring device comprising a water tank holding a prescribed amount of liquid, a stirring tower with an open top connected to the top of the water tank, the gas chamber being situated in the tank and having an open bottom through which the gas chamber is connected to the inside of the water tank and the stirring tower, and detecting means for generating control signals to close a valve to the methane gas outlet and open a valve to the methane gas inlet when detecting a prescribed upper level of the liquid rising in said stirring tower due to gas pressure in the gas chamber,
the treating tank being such that substances to be treated can be received at either the top or the bottom and treated substances can be discharged from the bottom or the top respectively.

The invention further provides a treating system for organic solid and liquid waste comprising:
a vertical alternate flow treating tank which comprises independent chambers connected to one another by connecting tubes wherein substances to be treated can flow alternately upward (or downward) and downward (or upward) and can be discharged from the final chamber,
filter media installed in each chamber in a standing position, the filter media being composed of a core element and a plurality of thin tapes of synthetic resin extending radially around the core element, the filter media being installed in such a manner that, in use of the system, the tapes float in liquid waste to be treated with their ends slightly touching those of adjacent tapes,
and an automatic stirring device having a gas chamber connected through valves to a methane gas outlet provided at an upper portion of the tank and to a methane gas inlet for stirring purposes at the bottom of the tank, the stirring device comprising a water tank holding a prescribed amount of liquid, a stirring tower with an open top connected to the top of the water tank, the gas chamber being situated in the water tank and having an open bottom through which the gas chamber is connected to the inside of the water tank and the stirring tower, and detecting means for generating control signals to close a valve to the methane gas outlet and open a valve to the methane gas inlet when detecting a prescribed upper level of the liquid rising in said stirring tower due to gas pressure in the gas chamber.

As indicated above, the filter medium placed in the chambers comprises a core element and a number of thin tapes of synthetic resin extending radially from the core element.

The treating system according to the invention includes an automatic gas-lift stirring device. The system preferably includes an automatic gas-lift stirring device having a gas tank connected through valves to a methane gas outlet provided at an upper portion of the tank and a methane gas inlet provided at the bottom of the tank. Said automatic gas-lift stirring device comprises a water tank which is filled with a prescribed amount of water and is connected to a stirring tower above, a gas chamber having an open bottom and placed in the water tank and a sensor which sends control signals to close a valve in a line to the methane gas outlet and open a valve in a line to the methane gas inlet by sensing when the water rises to a prescribed level in a stirring tower by gas pressure.

Any of the above treating tanks of horizontal zigzag flow type, vertical zigzag flow type and vertical alternate flow type is designed to have the fermentation treatment process take place in orderly fashion, by limiting direct delivery or useless circulation or detouring of the objects to be treated to a minimum and by making the whole process from inflow to discharge as even and as lengthy as practically possible. Thus, treatment efficiency per unit of hydraulic retention time (HRT) naturally increases.

A vertical alternate flow tank having air-tight independent chambers allows suspension of operation or repair of some chambers even while the rest of the system is in operation, directional inversion of the vertical flow in each chamber, or conversion of the chambers to other applications such as solid/liquid separation tanks or seeding tanks, by changing the connections of connecting tubes. It is also possible to manufacture each chamber as a standardized component through mass production, which will decrease manufacturing costs and lead to easier transportation, installation and repair.

Numerous filter media comprising a core element and thin tapes of synthetic resin extending radially from the core element are installed in the chambers of the treating tank in a standing or lying position, so that anaerobic bacteria are stored in large quantities in the tank. Said filter media move in an undulating manner in all directions at varying speeds by the effect of the automatic gas-lift stirring conducted in the tank, and therefore they are closer to a fluid-bed rather than a fixed bed so that the possibility of clogging is eliminated. Fluid-bed operation requires the maintenance of a good fluidity, so that the film of microbial membranes remains thin. The automatic gas-lift stirring controls gas pressure, gas volume and stirring frequency so that excess growth of microbial membranes is controlled to the optimal level. Accordingly the anaerobic treatment of industrial and home organic solid or liquid waste of low, medium or high content can be carried out with high efficiency.

By changing the location of the sensor and increasing or decreasing the amount of water in the water tank and the stirring tower in the automatic gas-lift stirring device, the volume and pressure of gas to be used for stirring and the intensity and frequency of stirring can be adjusted variously, so that optimal operation of the system is allowed in accordance with the amount of gas generated inside the treating tank. When gas generation increases in step with the increase in activity of methane-generating bacteria, the frequency of stirring also increases and thereby the activity of the methane-generating bacteria is accelerated

When the intensity(gas volume and pressure) and frequency of stirring exceed a peak, separation of the microbial membrane generated at the surface of the filter medium increases and the treatment efficiency drops.

However, with this automatic gas-lift stirring, the stirring frequency is automatically reduced as gas generation decreases after this peak and the separation is eliminated so that optimal gas generation is continuously maintained with stirring at the prescribed gas volume and pressure. Therefore, the stirring device is capable of maintaining the optimal level of activity of methane-generating bacteria in the treating tank as well as the optimal level of gas generation.

Accordingly, the treating system using the aforesaid treating tank, and further a treating system equipped with the treating tank and the automatic gas-lift stirring device is an optimal treating system for a wide range of organic solid and liquid waste which performs anaerobic treatment of industrial and home waste of low, medium or high content with high efficiency. In other words, there can be treated various kinds of organic waste, for example, treatment of flush toilet water which contains a relatively low concentration of organic substances, treatment of leftover food and kitchen waste water mixed with disposal-processed garbage which contains a relatively high concentration of organic substances, and treatment of a combination of part or all of these organic waste. It can also shorten the conventional treatment period of 20 to 40 days to one day or so. Adaptation of methane-generating bacteria to this system is easily made, and such problems as clogging and washout are also resolved. Thus the present invention provides the treating system which can perform anaerobic treatment of various organic solid and liquid waste such as domestic, livestock and industrial waste water, regardless of the concentration, type or scale thereof, with easy operation and high efficiency.

### BRIEF DESCRIPTION OF DRAWINGS

Fig. 1 is a schematic plan view of a horizontal zigzag flow tank showing one embodiment of the treating tank used in accordance with the present invention;
Fig. 2 is a schematic front view of the same;
Fig. 3 is a schematic plan view of a vertical zigzag flow tank showing another embodiment of the treating tank used in accordance with the present invention;
Fig. 4 is a schematic front view of the same;
Fig. 5 is a schematic plan view of the installation of gas flow pipes for stirring gas used with the horizontal zigzag flow tank;
Fig. 6 is a schematic plan view of the installation of gas flow pipes for stirring gas used with the vertical zigzag flow tank;
Fig. 7 is a plan view of a vertical alternate flow tank showing another embodiment of the treating tank used in accordance with the present invention;
Fig. 8 is a front view of the same;
Fig. 9 is a bottom view of the same;
Fig. 10 is a perspective view of a single unit of a vertical alternate flow tank which is assembled from component units;
Fig. 11 is a plan view of another embodiment of the vertical alternate flow tank showing a box type;
Fig. 12 is a front view of the same;
Fig. 13 is a bottom view of the same;
Fig. 14 is a perspective view of a single unit of a vertical alternate flow tank which is assembled from component units;
Fig. 15 is a schematic perspective view of the installation of heat exchange pipes connecting to each chamber of the vertical zigzag flow tank;
Fig. 16 is a schematic perspective view showing another embodiment of installation of said heat exchange pipes;
Fig. 17 and Fig. 18 are perspective views of the installation of heat exchange pipes used with the single unit of the assembly-type treating tank;
Fig. 19 is a front view of a treating system comprising a horizontal zigzag flow tank, automatic gas-lift stirring device and a gas tank in accordance with one embodiment of the present invention;
Fig. 20 is a front view showing one embodiment of filter medium to be installed in the treating tank;
Fig. 21 is a front view of a tape element of the filter medium;
Fig. 22 is a diagrammatic view showing the relationship between a core element and a tape element of the filter medium; and
Fig. 23 is a diagrammatic view of an entire treating system comprising a horizontal zigzag flow tank, an automatic gas-lift stirring device, a gas tank and a methane gas cleaning and purifying apparatus: for clarity, the filter media are not shown.

### BEST MODE FOR CARRYING OUT THE INVENTION

Referring to the attached drawings methane fermentation treatment of organic solid and liquid waste in accordance with the present invention is now described, but a filter medium and treating tank using said filter medium may be also applied to aerobic treating tanks and anaerobic fixed filtering beds.

### 1. Treating tanks

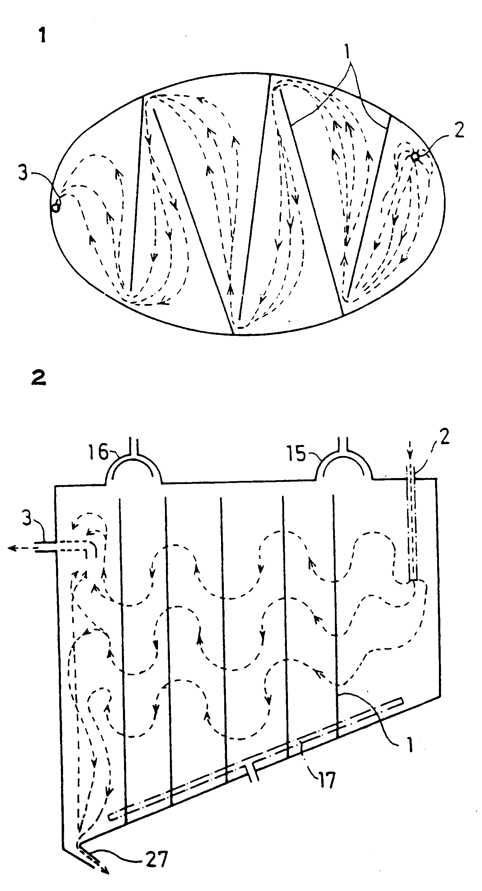

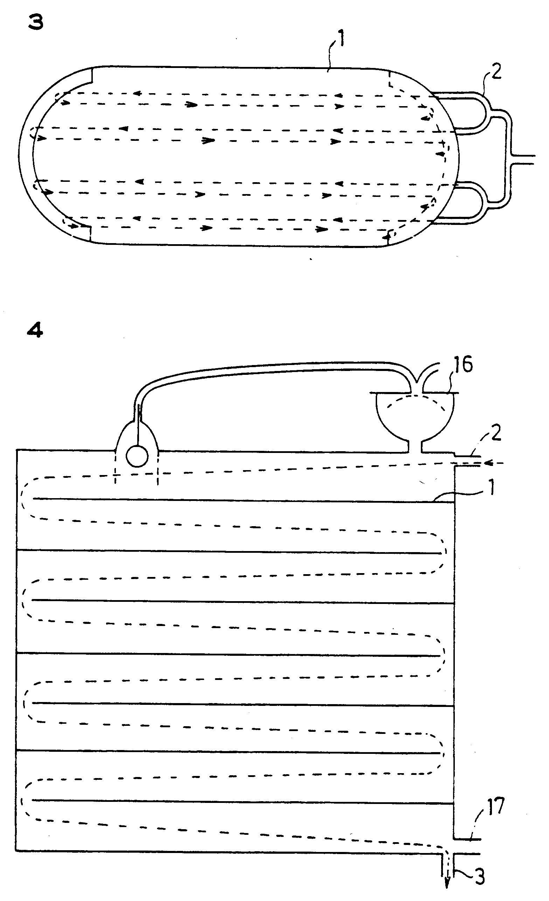

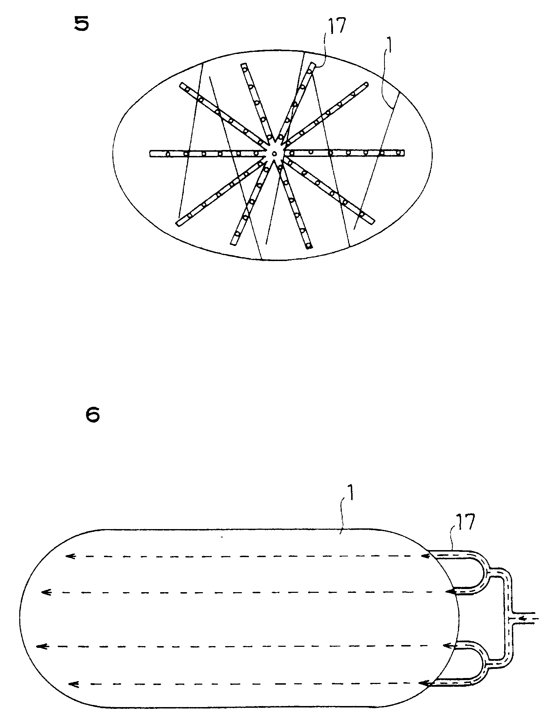

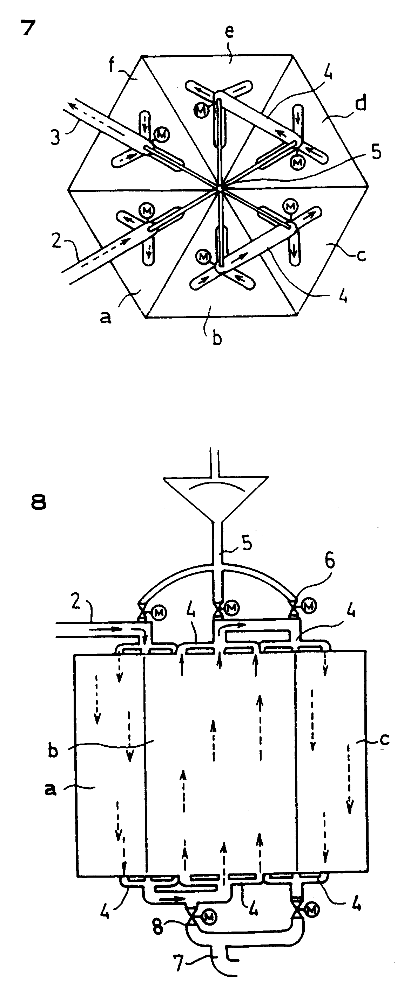

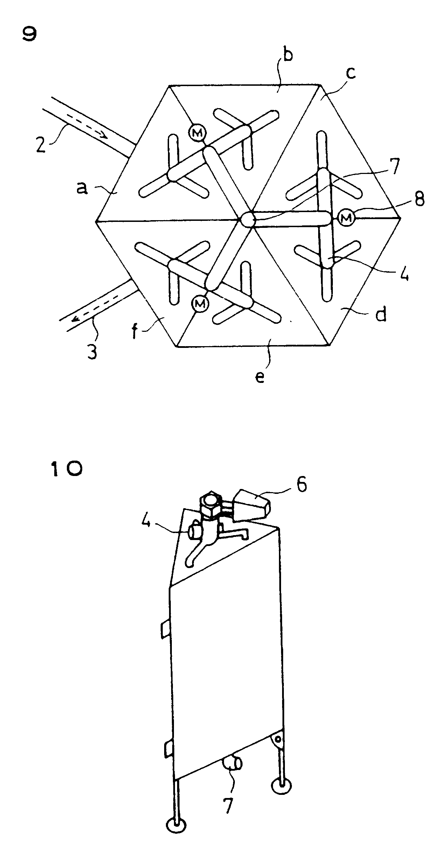

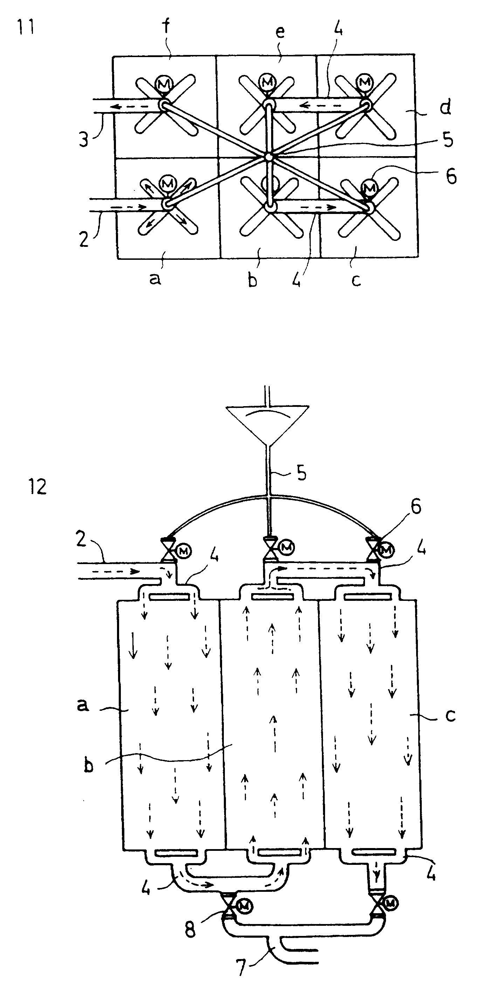

Figs. 1 through 6 represents embodiments of the treating tanks used in accordance with this invention. Figs. 1 and 2 show a horizontal zigzag flow tank and Figs. 3 and 4 show a vertical zigzag flow tank.

Although the tanks are represented as elliptic cylinders in the figures, various shapes including round or rectangular cylinders are employable depending on the location of installation, as far as organic substances are effectively treated in the course of horizontal zigzag flow or vertical zigzag flow inside the tank.

The horizontal zigzag flow tank in Figs. 1 and 2 has a sloped bottom and six to eight chambers partitioned by five to seven partitions 1. Organic substances introduced through an inlet 2 formed at the upper end of the slope reach the final chamber after completing the treatment process in a horizontal zigzag fashion and are discharged as treated liquid through an outlet 3 formed at the lower end of the slope, as shown in dotted lines.

In the vertical zigzag flow tank shown in Figs. 3 and 4, organic substances introduced through the inlet 2 reach the final chamber after completing the treatment process in a vertical zigzag fashion from top to bottom along partitions 1 which divide the tank horizontally, and are discharged through the outlet 3, as shown in dotted lines.

The number of partitions 1 is not limited. It has been proven that zigzag flow around the minimum two partitions and through three chambers shows a considerably high BOD (biochemical oxygen demand) removal rate.

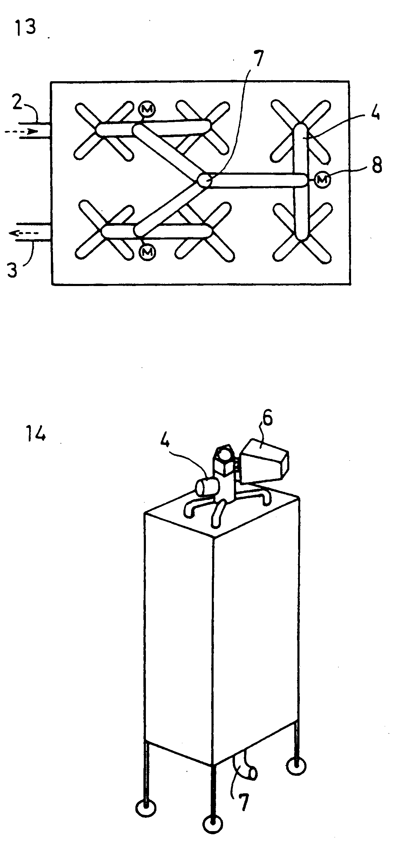

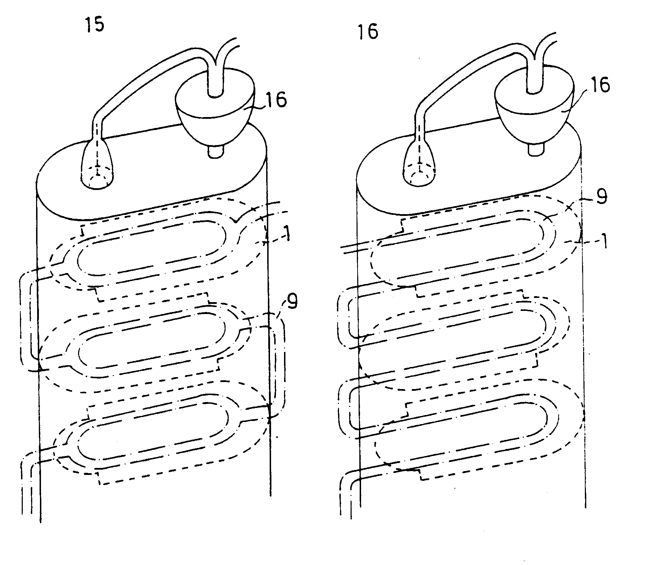

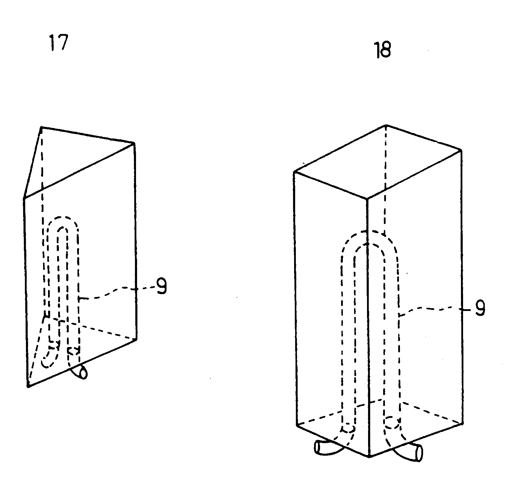

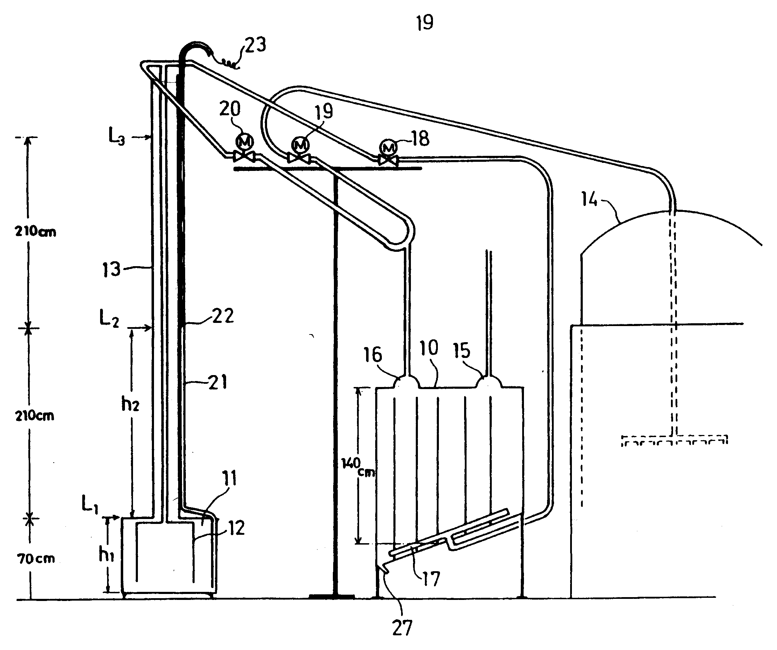

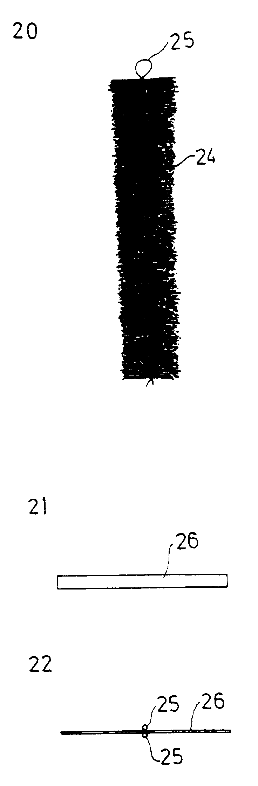

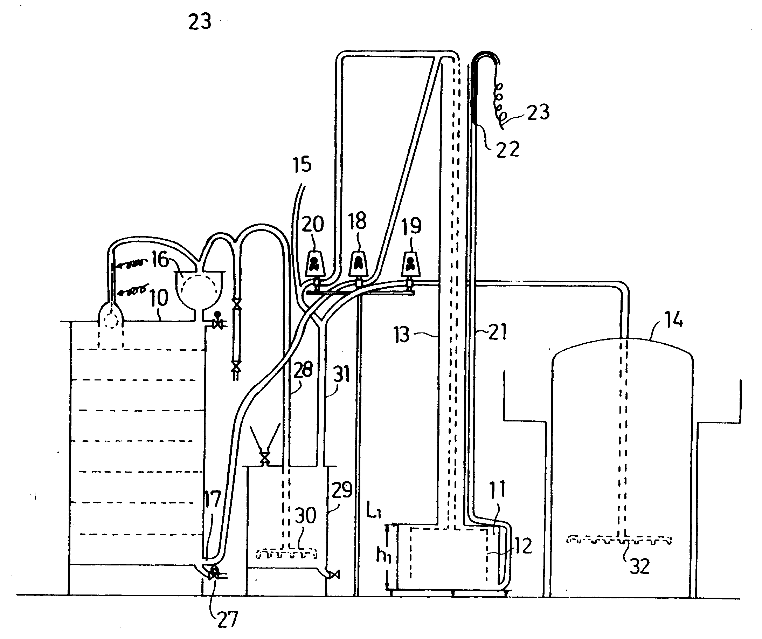

Figs. 7 through 14 represent a vertical alternate flow tank exemplifying another embodiment of the treating tank used according to this invention. Figs. 7 through 10 show a tank with a hexagonal cylindrical shape and Figs. 11 through 14 show a tank with a rectangular cylindrical shape. Other cylindrical shapes such as round or pentagonal shapes are also available.

Said tank may be constructed by equally dividing the inside thereof by partitions into six independent air-tight chambers or assembling six independent components of identical size and shape.

In the figures, there is shown by the dotted lines the process in which organic substances fed into the upper part of chamber a through an inlet 2 reach the bottom via downward flow, flow into the bottom of the next chamber b through a connecting tube 4, reach the upper part of chamber b via an upward flow, flow into the upper part of next chamber c through a connecting tube 4, reach the bottom of chamber c via a downward flow, repeating the same alternate upward and downward flows in chambers d, e and f, and reach the upper part of the chamber f. The treated organic substances are discharged through an outlet 3. If the first intake is at the bottom of the first chamber, the flow in the next chamber becomes a downward flow.

Figures 10 and 14 are perspective diagrams of embodiments of a single unit of an assembly-type treating tank. The unit in Figure 10 forms a hexagonal cylindrical tank, while the unit in Figure 14 forms a rectangular cylindrical tank.

In Figures 7 through 14, 5 is a methane gas outlet connected to each chamber via a motor-driven valve 6, and 7 is an inlet for methane gas used for stirring which is connected with each chamber via a motor-driven valve 8.

Figures 15 and 18 indicate the installation of heat exchange pipes in the tank. Heating of the horizontal zigzag flow tank can be achieved merely by heating the bottom of the tank, as convection can be used for the conduction of heat. But in the case of the vertical zigzag flow tank, as indicated in Figures 15 and 16, pipes 9 are installed in each stage and connected to each other in turns via connectors along the side or sides of the tank so that warm water is circulated therein. In the case of the assembly-type tank, as shown in Figures 17 and 18, a heat exchange pipe 9 is installed in each unit and connected with similar pipes in adjacent units via connectors so that warm water is circulated therein.

As mentioned above, the treating tank used in accordance with this invention, in each of three types (the horizontal zigzag flow tank, the vertical zigzag flow tank and the vertical alternate flow tank), is designed so that the treatment process is carried out in an orderly fashion by limiting the direct delivery, useless circulation or detour of organic substances to a minimum and by making the whole process from the intake to discharge as lengthy as practically possible. Therefore, the treatment efficiency per unit of hydraulic retention time naturally increases. While the reaction rate of a conventional treating tank in a single-tank system is low and it is said to take 20 to 40 days to complete the reaction, a horizontal zigzag flow tank with five chambers and four partitions is capable of achieving the same treatment results in five to ten days, a quarter of the time required for the conventional single-tank system even without installing filter media.

When an experiment was performed with installed filter media as described later, a horizontal zigzag flow tank (with four partitions and five chambers) could accomplish the treatment in one to two days, and the vertical zigzag flow tank could attain even higher efficiency.

Since the vertical alternate flow tank is composed of independent air-tight chambers, it is possible to suspend the operation of, or perform repairs to, some of the chambers during the operation of the system, change the direction of flow in any one chamber, or convert some chambers into different uses, such as solid/liquid separation tanks and seeding tanks, simply by changing the connection of connecting tubes 4. Although this type of tank involves high cost because a larger number of motor-driven valves and connecting tubes are required than in the horizontal zigzag flow tank or the vertical zigzag flow tank, the reduction of cost may be achieved by standarizing and massproducing the components. This also makes transportation and installation easier, which may compensate for the cost problem.

### 2. Automatic Gas-Lift Stirring Device

Figure 19 shows an embodiment of the treating system of this invention using one of the above tanks. In this figure, 10 is a horizontal zigzag flow tank with a capacity of 5m³. The same principle applies to a vertical zigzag flow tank or a vertical alternate flow tank as well. 11 is a 2m³ water tank having a 1m³ gas chamber 12 therein, to the top of which a stirring tower 13 is connected. 14 is a water-sealed gas tank.

The methane gas generated in the treating tank 10 gushes through methane gas outlets 15 and 16 provided at the top of the tank at equal pressure. Methane gas outlet 15 is connected to a pressure gauging and a safety device, for example such a device as disclosed in JP-A-63235839. Methane gas outlet 16 is connected to an automatic gas-lift stirring device as described below. Methane gas outlet 5 in the vertical alternate flow tank in Figure 7 through 14 is equivalent to this outlet 16 and is also connected to an automatic gas-lift stirring device. The water-sealed gas tank 14 does not exceed a prescribed gauge pressure. For example, when the gauge pressure is prescribed as being 0 to 0.13 atm (0 to 13.2 kPa), surplus methane gas escapes from the opening between the gas tank and the water tank when the tank is filled with methane gas with 0.13 gauge pressure, and the gauge pressure in the tank does not exceed 0.13 atm.

At the bottom of treating tank 10 is attached an intake pipe 17 for the stirring gas. As shown in Figure 5, this intake pipe has a plurality of branch pipes radially extending at even distances at the bottom of the tank, and each pipe has a number of small holes for blowing the gas into the tank. The number of holes is proportional to the area of the bottom of the tank. In the case of the vertical zigzag flow tank or the vertical alternate flow tank, the number of holes is proportional to the area of the bottom of each chamber or unit. It corresponds roughly to the number of core elements of the filter media or one per 15-30cm².

Outlet 16 of the treating tank is connected to the stirring tower 13 and the gas tank 14 via three motor-driven valves 18, 19 and 20 for controlling the automatic gas-lift stirring device. As described above, the stirring tower 13 is mounted on the 2m³ water tank 11 equipped with a 1m³ gas chamber 12 having the bottom open. The difference in capacity between water tank 11 and gas chamber 12 is 1m³ as well. The inner volume of the cylinder extending from point L₁ at the bottom to the level L₂ of the stirring tower 13 is also 1m³.

In order to measure the water level in the water tank 11 and the stirring tower 13, a transparent tube 21 having an opening in the vicinity of the tank bottom is installed along the water tank and the stirring tower. A control sensor 22 is inserted into the tube from the upper end thereof to perform automatic gas-lift stirring under various conditions. This sensor is fixed at the level L₂. 23 is a lead wire. Since strict precision is not required, the inner volume of the transparent tube will be disregarded below.

When 2m³ of water (or preferably anti-freeze solution) is poured from the upper end of stirring tower 13, the water fills up the gas chamber 12 as well as the water tank 11 through the open bottom of gas chamber 12, reaching the level L₁ which is the bottom level of the stirring tower 13. Methane gas generated in the treating tank 10 rises and gushes out from the outlet 16. When valve 20 is open among the three motor-driven valves 18, 19 and 20, the gas passes through valve 20 and enters the gas chamber 12, and thereby the water in the gas chamber 12 is expelled and rises through the opening between the water tank and the gas chamber into the stirring tower 13. Since methane gas continues to be generated in the treating tank 10, the volume and pressure of gas inside the gas chamber 12 increases, and thereby the water in the gas chamber rises gradually in the stirring tower 13. When the water level reaches the level L₂, the water level in transparent tube 21 reaches the same level as well and the sensor set at that level detects the water surface and sends out signals to be explained below.

When the water level reaches the level L₂, the gas chamber 12 is filled with 1m³ of methane gas and the gauge pressure therein is (h₁+h₂)cm/1,000cm, wherein h₁ is the depth of the water tank 11 and h₂ is the distance between L₁ and L₂. In case h₁ is 70cm and h₂ is 210cm, the gauge pressure is 0.28 (280cm/1000cm). The gauge pressure of the methane gas in the treating tank 10 is 0.28 as well.

At the instant that the sensor 23 detects the water at the level L₂, a signal is sent out to close the motor-driven valve 20 and open the valves 18 and 19. When the valve 19 is open, the methane gas of 0.28 atm (28.4 kPa) gauge pressure in the treating tank flows out to the gas tank 14 through the motor-driven valve 19 and the pressure in the tank decreases. Since motor-driven valve 18 is open, the 1m³ gas having 0.28 atm gauge pressure in the gas chamber 12 is led through the pipe via the motor-driven valve 18 to the treating tank and gushes as a stirring gas from a plurality of small holes of the intake pipe 17 branched at the bottom of the treating tank. The gushing gas rises in the treating tank, flowing from side to side and front to back between the filter media spreading full and wide in the tank with the core element in a standing position, thereby causing sufficient stirring, and then leaves the treating tank, flowing into the gas tank 14 via outlet 16 and motor-driven valve 19. The water depth at the bottom of the treating tank exerts a pressure against the intake of the stirring gas. Where the water depth is 140cm, the gauge pressure is 0.14 atm (14.2 kPa) which is higher than the highest gauge pressure, 0.13 atm (13.2 kPa) of the gas tank and the stirring gas no longer flows into the gas tank.

In this embodiment, the water column in the stirring tower 13 falls from a level of 280cm to 140cm, reducing the gas pressure from a gauge pressure of 0.28 atm to 0.14 atm (28.4 to 14.2 kPa). 0.5m³, that is half of the 1m³ is used for stirring and the remaining 0.5m³ remains in the gas chamber 12 with a gauge pressure of 0.14 atm (14.2 kPa).

When stirring is completed, the motor-driven valve 20 is opened and the valves 18 and 19 closed. The methane gas generated in the treating tank flows again into the gas chamber 12 and raises the water level in the stirring tower 13. Unless the amount of the gas generated in the treating tank changes, the water reaches the level L₂ in half the previous time and stirring starts. When the gas in the gas chamber 12 flows into the treating tank via the motor-driven valve 18, thereby performing stirring, and flows into the gas tank 14 via valve 19, the pressure levels in the gas chamber 12 and in the treating tank 10 attain equilibrium. Thereupon immediate reversion is made, that is, the motor-driven valve 20 is opened and the valves 18 and 19 are closed, and thereby the gas begins to accumulate again in the gas chamber 12 so that the preparation for yet another stirring is made. To artificially reduce the frequency of stirring, reversion is deferred after equilibrium is reached.

In the above example, the water level is originally maintained at L₁ by using 2m³ of water. If the water level is maintained at L₂ by using 3m³ of water and the sensor is set at L₃, 210cm higher than L_{2,} the pressure exceeds the gauge pressure of 0.14 (14.2 kPa) induced by 140cm depth of the treating tank and it is no longer necessary to be concerned with the water depth in the treating tank. When the water level reaches L₃ and stirring starts, all of the 1m³ of methane gas with gauge pressure of 0.21 to 0.49 atm (21.3 to 49.6 kPa) is used for stirring.

Except for cases where the gas in the gas chamber 12 escapes from the stirring tower into the atmosphere without being used for stirring prior to detecting the water level because the amount of water is insufficient or the sensor is located too high, when the location of the sensor is raised while the water level remains constant, the pressure and amount of gas used for stirring increases in general and stirring intensity becomes high, while stirring frequency decreases. Conversely, when the location of the sensor is lowered, the pressure and amount of gas decreases and stirring intensity becomes low while stirring frequency increases.

When the location of the sensor remains constant and the amount of water is increased, while the maximum gas pressure used for stirring remains the same, gas volume decreases, stirring intensity is generally high, and stirring frequency increases. Conversely, when the amount of water is reduced, while the maximum gas pressure remains the same, the gas volume increases, stirring intensity is low in general and stirring frequency decreases.

Thus by changing either the location of the sensor or the amount of water in the water tank and the stirring tower, the pressure and volume of the stirring gas and the stirring frequency may be varied, which permits the optimal operation of the system depending on the amount of gas generated in the treating tank.

### 3. Cleaning and purification of Methane Gas

As explained in section "2. Automatic Gas-lift stirring Device" above, the methane gas generated in the treating tank 10 comes into contact with the water in the gas chamber 12 and the stirring tower 13, and it is also violently mixed with the water in the gas chamber 12 and the stirring tower 13 during stirring process before it reaches the bottom 17 of the treating tank 10, where it forms bubbles rising through the filter media set in the water to be treated passing through the filter media, and finally flows into the gas tank 14 via outlet 16 and motor-driven valve 19.

Gas tank 14 is a water-sealed tank. The methane gas inlet pipe reaches the bottom of the tank, where the pipe end 32 branches out into a number of arms. Gushing gas forms bubbles rising up in the water and accumulates in the upper portion of the tank. Here again the methane gas is cleaned by the water.

Generally speaking, the gas generated from conventional methane gas generators is composed of 60-70% methane gas and 30-40% carbon dioxide. It may also contain a very small amount of hydrogen sulfide, depending on the kind of the organic substance to be treated. Therefore, although it is usable as fuel, it emits a slight odor during combustion and can be used only with a municipal gas combustion appliance.

The concentration of methane contained in the generated gas can be increased to 80-88% by the cleaning effect attained by passing the gas repeatedly into the water in the stirring process and in the gas tank, as mentioned above.

Of course, the concentration of methane varies depending on the kind and concentration of the organic substances to be treated, but the concentration of methane is approximately 20% higher (in other words, removing the corresponding concentration of carbon dioxide) than conventional methane gas generators. Therefore, the methane gas generated by this system emits no odor during combustion, and the calorific value is remarkably higher than municipal gas and the combustion efficiency is close to that of natural gas.

The gas generated vigorously in the treating tank contains many viscous particles. It is therefore necessary to install strainers at strategic points to prevent blockage of narrow spaces in the system. The particles remaining after passing through the strainers can be most effectively removed through water-cleaning.

Figure 23 represents an embodiment of the system of this invention comprising a vertical zigzag flow tank, an automatic gas-lift stirring device and a cleaning and purifying device 29 for the generated gas.

Referring to Figure 23, operation of the system is explained. The device 29 is filled with a saturated solution of calcium hydroxide Ca(OH)₂. Calcium hydroxide, or limewater, combines with CO₂ and precipitates as calcium carbonate.

The methane gas generated in the treating tank 10 enters the cleaning and purifing device 29 through outlet 16 and inlet pipe 28. The end 30 of pipe 28 branches at the bottom of the device 29, and the gas forms numerous bubbles rising up in the device and enters the gas chamber through pipe 31 and motor-driven valve 20 which is open.

After that, the process is the same as that explained with reference to Figure 19. Sensor 23 detects the water and closes the valve 20 and opens the valves 18 and 19. The gas entering the treating tank 10 via valve 18 and pipe 17 rises up from the bottom of the tank to the top in a zigzag fashion along and between the partitions, which process naturally exerts a significant cleaning effect.

The rising gas is sent to the cleaning and purifying device 29 through the outlet 16 and passes through the limewater again, and thereafter enters the gas tank through the valve 19 and the branched holes 32 at the bottom and rises to the top whereby the purification of the gas is repeated.

Therefore, the installation of the device 29 adds a total of two gas-cleaning processes by the limewater, one occurring before and one after automatic gas-lift stirring. Replacing the water in the water tank 12 and the gas tank 14 with limewater will enhance the purification of the methane gas. The resulting gas will be closer to natural gas. An experiment has proven that hydrogen sulfide is also removed. The precipitate, calcium carbonate or (Ca CO₃) can be treated and reused in the system. Mixing with a fertilizer enhances the effect of the fertilizer.

### 4. Filter Media

Each chamber of the treating tank is filled with a filter medium 24 as shown in Figure 20. A filter medium comprises a core element 25 and a number of thin tapes 26 made of synthetic resin extending radially around the core element. For example, the filter medium is made by aligning tapes 26 made of thin and wide synthetic resin film and cut to identical lengths of 30 to 60cm, at the same close intervals, placing stainless steel core elements 25 on both sides of the tapes at the center portion, and twisting core elements 25. While the method of manufacture is the same as for stick brushes, the appearance looks like a duster for automobiles which are made of long ostrich tail feathers. The filter media 24 are installed so as to fill each chamber of the treating tank in either a standing or lying position.

In one method of installation, for example, a number of filter media are attached to a frame at both ends of the core element 25 and said assembly is placed in each of the chambers. The filter media should be separated from each other at a distance such that the ends of the tapes 26 extending horizontally in the water to be treated slightly touch with those of adjacent tapes, because this degree of density permits easy operation and few breakdowns of the filter medium and also an effective treatment is obtainable.

Polypropylene is the best material for the filter medium. However, it is not necessary to limit to this material. Any material can be used as long as it has the same properties as polypropylene.

Polypropylene is obtainable stably and inexpensively. It is also light, with a specific gravity of 0.9. The tapes made of wide polypropylene films spread out straight to the end in the water to be treated. The material is very strong in physical property and no change of properties occurs in water. Because the material does not contain plasticizer, toxic or organic substances are never eluted in water.

Further, molecules being properly aligned lengthwise, the tape will not be severed transversely and shortened. In case strong shocks or vibrations are inflicted continuously over a long period of time, the tapes will simply split lengthwise into threads, and thereby the surface area is enlarged. Therefore, the more they are used, the more contact with bacteria they will have and the more sludge they will hold. As a result, the efficiency of methane fermentation is enhanced. Being entirely submerged in the water to be treated, the stainless steel core elements will not corrode.

The purpose of filling the treating tank with filter media or carriers is to hold as many reaction-causing anaerobic bacteria as possible by adhering the microbial membrane of the bacteria to the filter media or carriers. This principle applies to both the fixed-bed method and the fluidized bed method. With the fixed-bed method, however, the possibility of clogging is generally high, and therefore it is essential to deal with this problem.

The filter medium is used in this invention may be classified as a fixed-bed method. However, in view of its shape and function, and further in relation to the aforementioned automatic gas-lift stirring, it goes without saying that it does not entail the problem of clogging. The microbial membrane adhered to the tapes which spread in the treating tank moves in all directions at varying speeds by the action of the methane gas gushing out from the holes at the bottom of the tank, so the system is closer to a fluidized bed than a fixed bed. Fluidized bed is necessary to maintain good fluidity and keep the build up of microbial membrane relatively thin at all times. This requires the intake of organic substances and fluidity inside the tank through stirring. The filter media and the gas-lift stirring system in this invention are optimal to satisfy such requirements.

While it is generally said that the fluidized bed system is suitable for treatment of waste water having low concentrations of organic substances, this system can handle waste water of high, medium and low concentrations, because the aforementioned special filter media, as well as the ability to control the excess build up of microbial membrane through the control of the volume and pressure of the gas and the frequency of stirring, ensure optimal conditions.

Although it is said that the fluidized bed system is high in energy cost for creating fluidity in the treating tank, the automatic gas-lift stirring does not require any energy other than electric power for controlling the motor-driven valves because it utilizes the volume and pressure of gas generated in the treating tank.

### 5. Automatic Gas-Lift stirring Device and Filter Media

The microbial membranes adhered to the tapes 26 of the filter media move in all directions at varying speeds each time organic subtance is poured in and stirring takes place. As a result there occurs frequent and repeated collision between the membranes and the organic substances acting as substrate, so that the treating efficiency is increased. However, if the microbial membranes are separated to a large extent from the tapes by the shearing stress caused by the movement in the fluid and flow out, treating efficiency drops remarkably.

Conversely, if the microbial membranes settle firmly onto the tapes and begin to grow larger, it is said that the substrate penetrates into the inside of the membrane by molecular diffusion because there is no flow of fluid in the membrane and that there occurs a distribution of the substrate in the microbial membranes when the substrate is consumed by the microorganism in the diffusion process so that the treating efficiency decreases.

When the microbial membranes are separated to a large extent, it takes a considerable length of time for recovery because the membranes are created very slowly. Therefore, it is required that the separation is controlled so as not to form surplus microbial membranes so that the concentration of bacteria in the treating tank is maintained high at all times for a long period of time.

Control of membrane thickness has conventionally been performed by mechanical stirring or pumps, but it requires great skill and long experience and is still ambiguous. Recent microbiological research on anaerobic treatment has progressed greatly and enormous reports have been presented. Nevertheless, no method to measure the bacteria for the anaerobic treatment has been discovered. Even high-efficiency methane fermentation methods are not designed based on the metabolic or biological features of acid-generating bacteria and methane-generating bacteria, nor has any manipulation of such a nature been performed. Search and developments have been directed exclusively to maintaining as many anaerobic bacteria as possible in the treating tank. Accordingly treatment is limited to specific organic substances having high concentrations.

In order to treat organic substances of various kinds and of a wide range of concentrations, this invention provides the treatment tank of a horizontal zigzag flow, vertical zigzag flow or vertical alternate flow type, and at the same time, utilizes the generally-known fact that the degree of activity of methane-generating bacteria increases in proportion to the amount of methane gas generated. Of course such exceptions are excluded as that gas generation temporarily increases immediately after an excessive load is subjected accompanying the increase of the proportion of carbon dioxide and thereafter the activity of methane-generating bacteria and gas generation decreases significantly. When a certain amount of methane gas generated at a certain degree of activity of the methane-generating bacteria is used for stirring at a given pressure and frequency, the collision frequency between the microorganism and the organic substances increases, and the gas generation and the frequency of gas-lift stirring under the same gas volume and pressure increases. The methane-generating bacteria then become even more active and the gas generation and stirring frequency increases.

When the intensity (gas volume and pressure) and frequency of stirring exceeds a certain limit, separation of the microbial membranes increases and thereby the gas generation decreases. It is too late to alter the operation of the system after such decrease occurs, and restoration of the membranes will take a long time. In the gas-lift stirring in this invention, however, the gas generation decreases immediately after a peak is reached. Accordingly, stirring frequency is reduced and the separation is controlled and thus automatic control is attained so as to maintain optimal gas generation by stirring with a prescribed gas volume and pressure. This maintains the activity of the methane-generating bacteria in the treating tank and keeps gas generation at the maximum level under the preset conditions. In the operation of this system, the maximum gas volume (gas volume x gas pressure x stirring frequency) should be obtained by taking into consideration the nature, concentration and volume of the organic substances to be treated and changing the volume of water to be poured into the water tank and the stirring tower, as well as the location of the sensor, to ensure optimal efficiency in the treatment of the organic substance in the treating tank at that time.

### 6. Collecting and Removing Device for Scum and Sludge

Since the vertical alternate flow system performs alternate upward flow and downward flow and said flows are changeable via connecting tubes, there is no need to remove the scum which could accumulate in the upper portion of the treating tank and accumulation of sludge at the tank bottom can be prevented as well. But in the case of the horizontal zigzag flow system, there is a possibility that scum accumulates in the upper portion and sludge accumulates in the lowest portion of the tank.

Therefore, either inside or outside the tank wall, there is installed a jacket having a sloped bottom and an open top which is connected to the inside of the tank. Overflowing scum is collected in this jacket and removed through a pipe connected to it at the lowest end of the slope. Thus the scum can be effectively removed.

For removal of sludges contained in the waste water, it may be effective to install a sludge tank. Said sludge tank is connected via a valve to the lowest portion of the treating tank bottom. A float is provided in the sludge tank for detecting the surface of the liquid and closing the valve automatically when a certain amount of sludge has flowed into the sludge tank. The sludge in the sludge tank is sent back to the treating tank via a pump according to a predetermined cycle. In Figures 2 and 4, the connecting outlet to the sludge tank is indicated by numeral 27.

### INDUSTRIAL APPLICABILITY

As explained above in detail, the treating system of this invention improves filter media, treating tank and automatic gas-lift stirring in the tank, and provides the best system for the anaerobic treatment of organic solid and liquid waste. Accordingly the present system has various advantages over conventional systems of the same nature, e.g. wide applicability of various organic substances, high treatment efficiency, low installing and running costs, and easy operation.

An anaerobic treatment system utilizing the vertical alternate flow tank permits the repair, renovation and suspension of operation of a part of the tank without stopping the operation of the whole system just by changing the connections of the connecting tubes. It also permits the conversion of a part or all of the tank into a separation or seeding tank, or even into an aerobic tank or a mere anaerobic filter bed, while retaining high treatment efficiency.

It is also advantageous in that if the chambers are mass-produced as standarized components, reduction of costs and easy transportation can be attained and further a wide selection can be made for the location of installation. One of the chambers can be used as a seeding tank to maintain in a hungry state a number of the same methane-generating bacteria, which makes it possible to cope with the great change in the organic substances and the load. When seasonal fresh products or fruits are processed into secondary products, organic substances to be treated yields temporarily but in a great deal. Easy transportation of the system and full applicability of the methane -generating bacteria maintained in the seeding tank enables the optimal treatment of the above organic substances.

Methane fermentation treatment systems have been seen in many places for the treatment of livestock waste. However, in spite of the fact that farm owners obtained subsidies for the installation of these treatment systems, they were closed down in a few years because of their high operating and repairing costs. Since the system of this invention has few mechanical parts and receives and discharges organic substances with only a single pump, installation, running and repairing costs are low. In case the assembly-type system is operated or two or three sets of the horizontal zigzag flow system or the vertical zigzag flow system are operated simultaneously, no breakage will occur for a long period of time as regular maintenance and repairs can be made on individual units without stopping the whole operation.

The treated or fermented solution generated after methane fermentation treatment can be used for the manufacture of conventional compost. If appropriately diluted by water, it can also be used as an organic fertilizer having an immediate effect.

## Claims

1. A treating system for organic solid and liquid waste comprising:
a horizontal zigzag flow treating tank which comprises a sloping bottom and a plurality of chambers formed by horizontally dividing the inside of the tank with partitions disposed in a zigzag fashion with each partition oriented differently from an adjacent partition, the chambers being connected to one another in a horizontal zigzag fashion,
filter media to hold bacteria installed in each chamber in a standing position, the filter media being composed of a core element and a plurality of thin tapes of synthetic resin extending radially around the core element, the filter media being installed in such a manner that, in use of the system, the tapes float in liquid waste to be treated with their ends slightly touching those of adjacent tapes,
and an automatic stirring device having a gas chamber connected through valves to a methane gas outlet provided at an upper portion of the tank and to a methane gas inlet for stirring purposes at the bottom of the tank, the stirring device comprising a water tank holding a prescribed amount of liquid, a stirring tower with an open top connected to the top of the water tank, the gas chamber being situated in the water tank and having an open bottom through which the gas chamber is connected to the inside of the water tank and the stirring tower, and detecting means for generating control signals to close a valve to the methane gas outlet and open a valve to the methane gas inlet when detecting a prescribed upper level of the liquid rising in said stirring tower due to gas pressure in the gas chamber,
the treating tank being such that substances to be treated can be received at the upper end of the slope and treated substances can be discharged from the lower end of the slope.

2. A treating system for organic solid and liquid waste comprising:
a vertical zigzag flow treating tank which comprises a plurality of chambers formed by vertically dividing the inside of the tank with partitions extending in opposite directions in turn from the inner wall of the tank with a gap provided at each end so that the chambers are connected to one another in a vertical zigzag fashion whereby fluids to be treated flow substantially parallel to the partitions,
filter media to hold bacteria installed in each chamber in a lying position, the filter media being composed of a core element and a plurality of thin tapes of synthetic resin extending radially around the core element, the filter media being installed in such a manner that, in use of the system, the tapes float in liquid waste to be treated with their ends slightly touching those of adjacent tapes,
and an automatic stirring device having a gas chamber connected through valves to a methane gas outlet provided at an upper portion of the tank and to a methane gas inlet for stirring purposes at the bottom of the tank, the stirring device comprising a water tank holding a prescribed amount of liquid, a stirring tower with an open top connected to the top of the water tank, the gas chamber being situated in the water tank and having an open bottom through which the gas chamber is connected to the inside of the water tank and the stirring tower, and detecting means for generating control signals to close a valve to the methane gas outlet and open a valve to the methane gas inlet when detecting a prescribed upper level of the liquid rising in said stirring tower due to gas pressure in the gas chamber,
the treating tank being such that substances to be treated can be received at either the top or the bottom and treated substances can be discharged from the bottom or the top respectively.

3. A treating system for organic solid and liquid waste comprising:
a vertical alternate flow treating tank which comprises independent chambers connected to one another by connecting tubes wherein substances to be treated can flow alternately upward (or downward) and downward (or upward) and can be discharged from the final chamber,
filter media installed in each chamber in a standing position, the filter media being composed of a core element and a plurality of thin tapes of synthetic resin extending radially around the core element, the filter media being installed in such a manner that, in use of the system, the tapes float in liquid waste to be treated with their ends slightly touching those of adjacent tapes,
and an automatic stirring device having a gas chamber connected through valves to a methane gas outlet provided at an upper portion of the tank and to a methane gas inlet for stirring purposes at the bottom of the tank, the stirring device comprising a water tank holding a prescribed amount of liquid, a stirring tower with an open top connected to the top of the water tank, the gas chamber being situated in the water tank and having an open bottom through which the gas chamber is connected to the inside of the water tank and the stirring tower, and detecting means for generating control signals to close a valve to the methane gas outlet and open a valve to the methane gas inlet when detecting a prescribed upper level of the liquid rising in said stirring tower due to gas pressure in the gas chamber.

4. A system as claimed in any one of claims 1 to 3, which also comprises a purifying device for purifying gas issuing from the treating tank.

5. A system as claimed in any one of claims 1 to 4, wherein the water tank contains anti-freeze solution.

6. A method of treating liquid waste containing organic solid material, wherein a system as claimed in any one of claims 1 to 5 is used.

7. A method as claimed in claim 6, wherein the liquid waste contains a relatively low proportion of solid material.

8. A method as claimed in claim 6, wherein the liquid waste contains a relatively high proportion of solid material.

9. A method as claimed in any one of claims 6 to 8, wherein anaerobic treatment of the waste is effected.

## Patentansprüche

1. Behandlungssystem für festen und flüssigen organischen Abfall, umfassend
einen horizontalen Behandlungsbehälter mit Zickzackdurchfluß, der einen geneigten Boden und eine Vielzahl von Kammern umfaßt, die durch horizontales Unterteilen des Inneren des Behälters mit zickzackförmig angeordneten Zwischenwänden gebildet sind, wobei jede Zwischenwand unterschiedlich zur benachbarten Zwischenwand orientiert ist, wobei die Kammern miteinander in horizontaler Zickzackart verbunden sind,
Filtermedien, um Bakterien zu halten, die in jeder Kammer in stehender Stellung installiert sind, wobei die Filtermedien aus einem Kernelement und einer Vielzahl von dünnen Bändern aus synthetischem Harz, die sich radial um das Kernelement erstrecken, bestehen, wobei die Filtermedien in solcher Weise installiert sind, daß im Betrieb des Systems die Bänder im zu behandelnden flüssigen Abfall schwimmen, wobei ihre Enden diejenigen von benachbarten Bändern leicht berühren,
und eine automatische Rühreinrichtung, die eine Gaskammer besitzt, die über Ventile mit einem Methangasauslaß, der am oberen Teil des Behälters vorgesehen ist, und einem Methangaseinlaß für Rührzwecke am Boden des Behälters verbunden ist, wobei die Rühreinrichtung einen Wasserbehälter, der eine vorbestimmte Flüssigkeitsmenge enthält, einen Rührturm mit einem offenen oberen, mit der Oberseite des Wasserbehälters verbundenen Ende, wobei sich die Gaskammer im Wasserbehälter befindet und einen offenen Boden aufweist, durch den die Gaskammer mit dem Inneren des Wasserbehälters und des Rührturms verbunden ist, und Detektionsmittel zum Erzeugen von Steuersignalen zum Schließen eines Ventils zum Methangasauslaß und Öffnen eines Ventils zum Methangaseinlaß umfaßt, wenn ein vorbestimmter oberer Pegel von Flüssigkeit detektiert wird, die in dem Rührturm aufgrund des Gasdrucks in der Gaskammer ansteigt,
wobei der Behandlungsbehälter derart ist, daß zu behandelnde Substanzen am oberen Ende der Neigung aufgegeben und behandelte Substanzen vom unteren Ende der Neigung abgeführt werden können.

2. Behandlungssystem für festen und flüssigen organischen Abfall, umfassend
einen vertikalen Behandlungsbehälter mit Zickzackdurchfluß, der eine Vielzahl von Kammern umfaßt, die durch vertikales Unterteilen des Inneren des Behälters mit sich abwechselnd in entgegengesetzte Richtungen von der Innenwand des Behälters erstreckenden Zwischenwänden mit einem Spalt gebildet sind, der an jedem Ende vorgesehen ist, so daß die Kammern miteinander in vertikaler Zickzackart verbunden sind, wodurch zu behandelnde Flüssigkeiten im wesentlichen parallel zu den Zwischenwänden fließen,
Filtermedien, um Bakterien zu halten, die in jeder Kammer in liegender Stellung installiert sind, wobei die Filtermedien aus einem Kernelement und einer Vielzahl von dünnen Bändern aus synthetischem Harz, die sich radial um das Kernelement erstrecken, bestehen, wobei die Filtermedien in solcher Weise installiert sind, daß im Betrieb des Systems die Bänder im zu behandelnden flüssigen Abfall schwimmen, wobei ihre Enden diejenigen von benachbarten Bändern leicht berühren,
und eine automatische Rühreinrichtung, die eine Gaskammer besitzt, die über Ventile mit einem Methangasauslaß, der am oberen Teil des Behälters vorgesehen ist, und einem Methangaseinlaß für Rührzwecke am Boden des Behälters verbunden ist, wobei die Rühreinrichtung einen Wasserbehälter, der eine vorbestimmte Flüssigkeitsmenge enthält, einen Rührturm mit einem offenen oberen, mit der Oberseite des Wasserbehälters verbundenen Ende, wobei sich die Gaskammer im Wasserbehälter befindet und einen offenen Boden aufweist, durch den die Gaskammer mit dem Inneren des Wasserbehälters und des Rührturms verbunden ist, und Detektionsmittel zum Erzeugen von Steuersignalen zum Schließen eines Ventils zum Methangasauslaß und Öffnen eines Ventils zum Methangaseinlaß umfaßt, wenn ein vorbestimmter oberer Pegel von Flüssigkeit detektiert wird, die in dem Rührturm aufgrund des Gasdrucks in der Gaskammer ansteigt,
wobei der Behandlungsbehälter derart ist, daß zu behandelnde Substanzen entweder am oberen Ende oder am Boden aufgegeben und behandelte Substanzen vom Boden bzw. oberen Ende abgeführt werden können.

3. Behandlungssystem für festen und flüssigen organischen Abfall, umfassend
einen vertikalen Behandlungsbehälter mit wechselndem Durchfluß, der eine Vielzahl von Kammern umfaßt, die miteinander durch Verbindungsrohre verbunden sind, wobei zu behandelnde Substanzen abwechselnd aufwärts (oder abwärts) und abwärts (oder aufwärts) fließen und aus der letzten Kammer abgeführt werden können,
Filtermedien, die in jeder Kammer in stehender Stellung installiert sind, wobei die Filtermedien aus einem Kernelement und einer Vielzahl von dünnen Bändern aus synthetischem Harz, die sich radial um das Kernelement erstrecken, bestehen, wobei die Filtermedien in solcher Weise installiert sind, daß im Betrieb des Systems die Bänder im zu behandelnden flüssigen Abfall schwimmen, wobei ihre Enden diejenigen von benachbarten Bändern leicht berühren,
und eine automatische Rühreinrichtung, die eine Gaskammer besitzt, die über Ventile mit einem Methangasauslaß, der am oberen Teil des Behälters vorgesehen ist, und einem Methangaseinlaß für Rührzwecke am Boden des Behälters verbunden ist, wobei die Rühreinrichtung einen Wasserbehälter, der eine vorbestimmte Flüssigkeitsmenge enthält, einen Rührturm mit einem offenen oberen, mit der Oberseite des Wasserbehälters verbundenen Ende, wobei sich die Gaskammer im Wasserbehälter befindet und einen offenen Boden aufweist, durch den die Gaskammer mit dem Inneren des Wasserbehälters und des Rührturms verbunden ist, und Detektionsmittel zum Erzeugen von Steuersignalen zum Schließen eines Ventils zum Methangasauslaß und Öffnen eines Ventils zum Methangaseinlaß umfaßt, wenn ein vorbestimmter oberer Pegel von Flüssigkeit detektiert wird, die in dem Rührturm aufgrund des Gasdrucks in der Gaskammer ansteigt.

4. System nach einem der Ansprüche 1 bis 3, das auch eine Reinigungseinrichtung für Reinigungsgas umfaßt, das vom Behandlungsbehälter abgegeben wird.

5. System nach einem der Ansprüche 1 bis 4, wobei der Wasserbehälter Antifrostlösung enthält.

6. Verfahren zur Behandlung flüssigen Abfalls enthaltend festes organisches Material, wobei ein System wie in einem der Ansprüche 1 bis 5 beansprucht verwendet wird.

7. Verfahren nach Anspruch 6, wobei der flüssige Abfall einen relativ niedrigen Anteil an festem Material enthält.

8. Verfahren nach Anspruch 6, wobei der flüssige Abfall einen relativ hohen Anteil an festem Material enthält.

9. Verfahren nach einem der Ansprüche 6 bis 8, bei dem eine anaerobe Behandlung des Abfalls durchgeführt wird.

## Revendications

1. Système de traitement destiné aux déchets organiques solides et liquides, comprenant :
une cuve de traitement à circulation horizontale en zigzag, qui comprend un fond en pente et une pluralité de chambres formées en divisant horizontalement l'intérieur de la cuve à l'aide de cloisons disposées en zigzag, chaque cloison étant orientée différemment d'une cloison adjacente, les chambres étant reliées l'une à l'autre en zigzag suivant l'horizontale,
des milieux filtrants destinés à contenir des bactéries, installés dans chaque chambre en position debout, les milieux filtrants étant composés d'un élément central et d'une pluralité de minces rubans de résine synthétique qui s'étendent radialement autour de l'élément central, les milieux filtrants étant installés de telle manière que, pendant l'utilisation du système, les rubans flottent dans les déchets liquides à traiter, leurs extrémités touchant légèrement celles des rubans adjacents,
et un dispositif d'agitation automatique comportant une chambre à gaz reliée par l'intermédiaire de vannes à un orifice de sortie de gaz méthane disposé au niveau d'une partie supérieure de la cuve ainsi qu'à un orifice d'entrée de gaz méthane destiné à l'agitation au niveau du fond de la cuve, le dispositif d'agitation comprenant une cuve à eau contenant une quantité prescrite de liquide, une tour d'agitation comportant un sommet ouvert reliée au sommet de la cuve à eau, la chambre à gaz étant située dans la cuve à eau, et comportant un fond ouvert par l'intermédiaire duquel la chambre à gaz est reliée à l'intérieur de la cuve à eau et de la tour d'agitation, et un moyen de détection destiné à générer des signaux de commande afin de fermer une vanne de l'orifice de sortie de gaz méthane et d'ouvrir une vanne de l'orifice d'entrée de gaz méthane lors de la détection d'un niveau supérieur prescrit du liquide qui remonte dans ladite tour d'agitation en raison de la pression du gaz dans la chambre à gaz,
la cuve de traitement étant telle que les substances à traiter peuvent être reçues au niveau de l'extrémité supérieure de la pente et les substances traitées peuvent être évacuées depuis l'extrémité inférieure de la pente.

2. Système de traitement destiné à des déchets organiques solides et liquides comprenant :
une cuve de traitement à circulation verticale en Zigzag, qui comprend une pluralité de chambres formées en divisant verticalement l'intérieur de la cuve à l'aide de cloisons s'étendant dans des directions opposées tour à tour depuis la paroi intérieure de la cuve, un espace étant ménagé à chaque extrémité, de sorte que les chambres sont reliées l'une à l'autre suivant un zigzag vertical, grâce à quoi les fluides à traiter circulent de façon pratiquement parallèle aux cloisons,
des milieux filtrants destinés à contenir des bactéries, installés dans chaque chambre en position couchée, les milieux filtrants étant composés d'un élément central et d'une pluralité de minces rubans de résine synthétique qui s'étendent radialement autour de l'élément central, les milieux filtrants étant installés de telle manière que, pendant l'utilisation du système, les rubans flottent dans les déchets liquides à traiter, leurs extrémités touchant légèrement celles des rubans adjacents,
et un dispositif d'agitation automatique comportant une chambre à gaz reliée par l'intermédiaire de vannes à un orifice de sortie de gaz méthane disposé au niveau de la partie supérieure de la cuve ainsi qu'à un orifice d'entrée de gaz méthane destiné à l'agitation au niveau du fond de la cuve, le dispositif d'agitation comprenant une cuve à eau contenant une quantité prescrite de liquide, une tour d'agitation comportant un sommet ouvert, reliée au sommet de la cuve à eau, la chambre à gaz étant située dans la cuve à eau et comportant un fond ouvert par l'intermédiaire duquel la chambre à gaz est reliée à l'intérieur de la cuve à eau et de tour d'agitation, et un moyen de détection destiné à générer des signaux de commande afin de fermer une vanne de l'orifice de sortie de gaz méthane et d'ouvrir une vanne de l'orifice d'entrée de gaz méthane lors de la détection d'un niveau supérieur prescrit du liquide qui remonte dans ladite tour d'agitation en raison de la pression du gaz dans la chambre à gaz,
la cuve de traitement étant telle que les substances à traiter peuvent être reçues soit en haut, soit en bas, et les substances traitées peuvent être évacuées depuis le bas ou le haut, respectivement.

3. Système de traitement destiné à des déchets organiques solides et liquides comprenant :
une cuve de traitement à circulation verticale alternée, qui comprend des chambres indépendantes reliées l'une à l'autre par des tubes de raccordement dans lesquelles les substances à traiter peuvent circuler en alternance vers le haut (ou vers le bas) et vers le bas (ou vers le haut) et peuvent être évacuées à partir de la chambre finale,
des milieux filtrants installés dans chaque chambre en position debout, les milieux filtrants étant composés d'un élément central et d'une pluralité de minces rubans de résine synthétique qui s'étendent radialement autour de l'élément central, les milieux filtrants étant installés de telle manière que, pendant l'utilisation du système, les rubans flottent dans les déchets liquides à traiter, leurs extrémités touchant légèrement celles des rubans adjacents,
et un dispositif d'agitation automatique comportant une chambre à gaz reliée par l'intermédiaire de vannes à un orifice de sortie de gaz méthane disposé au niveau d'une partie supérieure de la cuve ainsi qu'à un orifice d'entrée de gaz méthane destiné à l'agitation, au fond de la cuve, le dispositif d'agitation comprenant une cuve à eau contenant une quantité prescrite de liquide, une tour d'agitation comportant un sommet ouvert, reliée au sommet de la cuve à eau, la chambre à gaz étant située dans la cuve à eau et comportant un fond ouvert par l'intermédiaire duquel la chambre à gaz est reliée à l'intérieur de la cuve à eau et de la tour d'agitation, et un moyen de détection destiné à générer des signaux de commande afin de fermer une vanne de l'orifice de sortie de gaz méthane et d'ouvrir une vanne de l'orifice d'entrée de gaz méthane lors de la détection d'un niveau supérieur prescrit du liquide qui remonte dans ladite tour d'agitation en raison de la pression du gaz dans la chambre à gaz.

4. Système selon l'une quelconque des revendications 1 à 3, qui comprend également un dispositif d'épuration destiné à épurer le gaz sortant de la cuve de traitement.

5. Système selon l'une quelconque des revendications 1 à 4, dans lequel la cuve à eau contient une solution antigel.

6. Procédé de traitement d'eaux usées contenant des matières organiques solides, dans lequel est utilisé un système selon l'une quelconque des revendications 1 à 5.

7. Procédé selon la revendication 6, dans lequel les eaux usées contiennent une proportion relativement faible de matières solides.

8. Procédé selon la revendication 6, dans lequel les eaux usées contiennent une proportion relativement élevée de matières solides.

9. Procédé selon l'une quelconque des revendications 6 à 8, dans lequel un traitement anaérobie des déchets est effectué.
